# FASCICULE DE BREVET EUROPEEN

(11) **EP 2 934 702 B1**
(45) Date de publication et mention de la délivrance du brevet: **17.01.2018**
(21) Numéro de dépôt: 13811898.9
(22) Date de dépôt: 18.12.2013
(51) Int. Cl.: A61Q 19/06, A61K 8/97, A61K 36/185, A61P 17/00, A61P 9/00

(54) **EXTRAIT DE GRAINES DE PASSIFLORE ET COMPOSITIONS COSMETIQUES, PHARMACEUTIQUES, DERMATOLOGIQUES OU NUTRACEUTIQUES LE COMPRENANT**
EXTRAKT AUS PASSIONSBLUMENSAMEN UND KOSMETISCHE, PHARMAZEUTISCHE, DERMATOLOGISCHE UND NUTRAZEUTISCHE ZUSAMMENSETZUNGEN DAMIT
EXTRACT OF PASSIONFLOWER SEEDS AND COSMETIC, PHARMACEUTICAL, DERMATOLOGICAL AND NUTRACEUTICAL COMPOSITIONS COMPRISING SAME

(30) Priorité: 18.12.2012 FR 1262234
(43) Date de publication de la demande: 28.10.2015
(73) Titulaire: Laboratoires Expanscience, 92048 Paris La Défense Cedex (FR)
(72) Inventeur: MSIKA, Philippe, F-78000 Versailles (FR); LECLERE-BIENFAIT, Sophie, F-28100 Dreux (FR); DEBROCK, Sébastien, F-28210 St Martin De Nigelles (FR); BREDIF, Stéphanie, F-28210 Croisilles (FR); GARNIER, Sébastien, F-06650 Le Rouret (FR)
(74) Mandataire: Regimbeau
(86) Numéro de dépôt international: PCT/EP2013/077065
(87) Numéro de publication internationale: WO 2014/095983

(56) Documents cités:
- JP-A- 2009 102 298
- US-A1- 2012 004 322

## Description

L'invention se rapporte à une composition comprenant un extrait de graines de Passiflore, *Passiflore incarnata* ou *edulis* et préférentiellement *edulis.* La composition est avantageusement cosmétique, pharmaceutique, dermatologique, nutraceutique. L'invention a également pour objet un procédé d'extraction d'un extrait de graines de Passiflore, ainsi que l'extrait susceptible d'être obtenu par ledit procédé. L'invention concerne également une telle composition ou un tel extrait pour son utilisation dans la prévention ou le traitement des troubles ou pathologies de la peau, des muqueuses ou des phanères, pour son utilisation dans la prévention ou le traitement des troubles vasculaires, pour son utilisation pour lutter contre le stress oxydant, ou encore pour son utilisation dans la prévention ou le traitement des altérations du tissu adipeux. L'invention concerne enfin un procédé de soin cosmétique de la peau, des phanères ou des muqueuses, en vue d'améliorer leur état ou leur aspect, consistant à administrer une telle composition ou un tel extrait.

### LES PASSIFLORES

La famille des passiflores (Passiflora) est constituée d'environ 500 espèces. Les espèces sont souvent distribuées dans les régions chaudes tempérées et tropicales et notamment sur le continent américain, mais sont plutôt rares en Asie, Australie et Afrique tropicale.

### Botanipue

Les plants se présentent sous forme d'arbrisseaux ou herbes grimpantes. Les feuilles sont alternes, parfois simples, lobées ou palmées. Les fleurs peuvent attendre 9 cm de diamètre sont bisexuelles ou unisexuelles et régulières. Elles sont blanches et violettes et présentent des appendices pétaloïdes fins, garnis d'appendices filiformes symbolisant la couronne d'épines du Christ. Le fruit de 4 à 5 cm de longueur est ovalaire et souvent de couleur jaune à orangé. Les espèces les plus répandues sont notamment *P. incarnata et P. edulis.*

### Eléments de Phytochimie

Les plus étudiées sont *P. incarnata* et *P. edulis.*

*P. incarnata :* les constituants majeurs sont représentés par la famille des flavonoïdes qui sont présentes en grande quantité dans les feuilles. Elle contient une forte teneur en isovitexine. Elle contient également en faible quantité des alcaloïdes indoliques simples (harmane, harmine...), des sucres comme le raffinose, le saccharose, fructose, glucose, et des huiles essentielles et du maltol décrit comme la molécule qui serait à l'origine des effets sédatifs et anti-convulsifiants attribués à cette plante.

*P. edulis :* à partir d'un extrait méthanolique de feuilles séchées a été identifié un composé spécifique, la passiflorine - cyclopropane triterpène glycoside (E. Bombardelli et al., 1975).

Elle contient de l'isoorientine, flavonoïde qui n'est pas retrouvée dans *P. incarnata* ainsi que des traces d'huile essentielle et d'alcaloïdes identiques à *P. incarnata.*

La pulpe du fruit contient des flavonoïdes, schaftoside, isoschaftoside, isoorientine, orientine, isovitexine, dérivés de lutéoline (M.L. Zeraik, J.H. Yariwake - 2010), de l'acide ascorbique (environ 60 mg/100 g).

La pulpe contient également des dérivés cyanogéniques glycosylés : la prunasine, la sambunigrine et l'amygdaline, ainsi que récemment identifiés, deux mandelonitrile β-rutinosides (D. Chassagne and J. Crouzet, 1998 ; D.S. Seigler, 2002).

### Toxicologie

Des constituants cyanogéniques sont présents essentiellement dans les parties aériennes de différentes variétés de passiflore.

### Caractéristipues de la graine

Les graines constituent 6 à 12% du fruit de *P. Edulis* et elles contiennent :
- *des polyphénols* dont le Piceatannol (structure proche du resveratrol) et son dimère la scirpusine B (S. Sano ; K. Sugiyama ; T. Ito, 2011), substances à effets vasorelaxant et anti-oxydant.
- *de l'huile,* 18% par solvant contenant des phytostérols (0.2% dont campestérol, stigmastérol, sitostérol, avenasterol) ; 60 à 73% d'acide linoléique (oméga 6), 14% à 20% d'acide oléique et 465 ppm de tocophérols (G. Piobom, N. Barou et al, 2006 ; R. de V.V. Lopes et al.).
- *Des sucres et des protéines*

### ART ANTERIEUR

### Usage Alimentaire

Le fruit semble être consommé depuis la préhistoire. Au XVIème siècle au Pérou, les magnifiques fleurs de passiflores étaient déjà considérées comme un remède et de nombreuses espèces de passiflores sont toujours utilisées dans de nombreux pays dans les pratiques thérapeutiques courantes.

### Usage Médical

Les passiflores (souvent les parties aériennes et parfois les fruits) sont souvent utilisées partout dans le monde comme anxiolytique, sédatif, diurétique et analgésique (toutes les descriptions dans « Passiflora : review update. K. Dhawan, S. Dhawan, A. Sharma, 2004 »). Le maltol et certains de ses dérivés seraient à l'origine de cet effet sédatif.

Cette activité serait plus constante et plus significative pour *P. incarnata.*

Des extraits de *P. incarnata* seraient capables de réverser la dépendance à la morphine.

Des effets anti-inflammatoires ont également été mis en évidence pour des extraits de feuilles de *P. edulis.*

Les différentes familles de polyphénols contribuent vraisemblablement de manière très importante à l'effet anti-oxydant et anti-glycation des protéines (M. Rudnicki et al, 2007) des parties aériennes de *P. edulis.*

Un effet anti-hypotenseur d'un extrait méthanolique d'écorces de fruits de *P. edulis* ainsi qu'un effet hypocholestémiant d'un extrait de graines délipidées riches en fibres ont également été mis en évidence.

Effet anti-tumoral de décoction de fruit via l'inhibition de métalloprotéinases matricielles (MMP2 et MMP9) impliquées dans l'invasion tumorale, les métastases et l'angiogénèse (S.S. Patel, 2009).

### Usage Dermo-cosmétiques

Usage cutané des feuilles de *P. foetida* au Brésil pour traiter les maladies cutanées d'origine inflammatoire. A Maurice et Rodrigues les décoctions de feuilles de *P. suberosa* sont utilisées en bain pour traiter les maladies de peau.

### DESCRIPTION DE L'INVENTION

La Demanderesse a découvert que les extraits de graines de passiflore, en particulier *Passiflore incarnata* ou *Passiflore edulis,* et encore plus avantageusement *Passiflore edulis,* présentent des propriétés cosmétiques et dermatologiques jamais décrites jusqu'à présent. En particulier, c'est la première fois que de tels extraits de graines de passiflore sont utilisés en tant que tels, pour leurs propriétés spécifiques.

L'invention a pour objet une composition comprenant un extrait peptidique et osidique de graines de passiflore, en particulier de graines de *Passiflore incarnata* ou de *Passiflore edulis,* avantageusement de *Passiflore edulis,* en tant que principe actif, et le cas échéant un excipient approprié, caractérisée en ce que l'extrait peptidique et osidique est obtenu par hydrolyse enzymatique en présence d'au moins une protéase et au moins une carbohydrase. La composition est avantageusement cosmétique, pharmaceutique, dermatologique. La composition peut aussi être nutraceutique. Ladite composition est de préférence formulée pour être administrée par voie topique externe ou orale.

Par « extrait peptidique et osidique », on entend un extrait comprenant majoritairement ou essentiellement des peptides et des oses (sucres). Les protéines naturellement présentes dans les graines ont été hydrolysées en peptides, avantageusement l'hydrolyse est une hydrolyse enzymatique.

Dans le cadre de la présente invention, l'extrait peptidique et osidique est avantageusement obtenu par hydrolyse enzymatique, plus avantageusement en présence d'au moins une protéase et au moins une carbohydrase. Il est plus avantageusement susceptible d'être obtenu par le procédé décrit plus loin dans la description.

Dans l'extrait peptidique et osidique, les peptides ont avantageusement une masse moléculaire inférieure à 3500 Da. Ces peptides recouvrent tous les composés à base d'aminoacides présents dans l'extrait.

Selon une variante avantageuse de l'invention, dans l'extrait peptidique et osidique, au moins 90% des peptides ont une masse moléculaire inférieure à 1200 Da.

Dans l'extrait peptidique et osidique, avantageusement au moins 30% des peptides, plus avantageusement au moins 40% des peptides, ont une masse moléculaire inférieure à 300 Da. Selon une variante avantageuse de l'invention, entre 30% et 50% des peptides ont une masse moléculaire inférieure à 300 Da.

Avantageusement selon l'invention, entre 30% et 70%, plus avantageusement entre 40% et 60%, des peptides de l'extrait ont une masse moléculaire comprise entre 300 et 1200 Daltons.

La répartition des masses molaires des peptides est exprimée en pourcentage par rapport à la concentration totale de peptides.

Typiquement, l'extrait peptidique et osidique selon l'invention ne contient substantiellement pas de protéines résiduelles qui sont potentiellement allergisantes.

L'extrait selon la présente invention comprend avantageusement 10 à 90 % en poids de peptides et 10 à 90 % en poids de sucres totaux, les pourcentages étant exprimés par rapport au poids total dudit extrait (avant ajout éventuel d'un support de séchage).

Dans l'extrait peptidique et osidique, le ratio massique peptides/sucres est avantageusement supérieur à 0,75 et de préférence compris entre 1 et 2

L'extrait peptidique et osidique comprend avantageusement 20 à 70 %, avantageusement 30 à 65 %, typiquement 55%, en poids de peptides, les pourcentages étant exprimés par rapport au poids total dudit extrait.

L'extrait peptidique et osidique comprend avantageusement 20 à 60 %, avantageusement 30 à 55 %, typiquement 40%, en poids de sucres, les pourcentages étant exprimés par rapport au poids total dudit extrait.

En particulier, l'extrait peptidique et osidique comprend avantageusement 20 à 70 %, avantageusement 30 à 65 %, en poids de peptides et 20 à 60 %, avantageusement 30 à 55 %, en poids de sucres, les pourcentages étant exprimés par rapport au poids total dudit extrait.

Selon un aspect préféré de l'invention, l'extrait peptidique et osidique comprend 55 % en poids de peptides et 40 % en poids de sucres, les pourcentages étant exprimés par rapport au poids total de matière active dudit extrait avant ajout par exemple d'un éventuel support de séchage. Les 5% restant sont des minéraux (cendres) et autres molécules diverses.

Selon une variante avantageuse de l'invention, la composition contient 0,001 à 10 %, typiquement 0,01 à 5 %, en poids d'extrait, exprimé en pourcentage d'extrait sec.

L'invention a également pour objet un procédé de préparation d'un extrait peptidique et osidique de graines de passiflore en particulier de graines de *Passiflore incarnata* ou de *Passiflore edulis,* avantageusement de *Passiflore edulis,* comprenant au moins une étape d'hydrolyse enzymatique en présence d'au moins une protéase et au moins une carbohydrase dans les conditions optimales de pH et de température, connues de l'homme de l'art. Le traitement enzymatique de la dispersion peut être suivi si besoin d'un traitement thermique afin de dénaturer les enzymes.

Avantageusement selon l'invention, le procédé de préparation d'un extrait peptidique et osidique de graines de passiflore comprend les étapes successives suivantes :
a) dispersion en phase aqueuse des graines broyées ; puis
b) traitement enzymatique de la dispersion aqueuse obtenue à l'étape a), puis
c) le cas échéant, traitement thermique pour dénaturer les enzymes, et
d) récupération de l'extrait peptidique et osidique à la fin de l'étape b) ou c).

A l'étape a), la phase aqueuse est avantageusement de l'eau.

Le traitement enzymatique (étape b) est avantageusement réalisé par ajout d'au moins une protéase et d'au moins une carbohydrase, dans les conditions optimales de pH et de température, connues de l'homme de l'art. Le pH est par exemple compris entre 3,0 et 9,0. La température est typiquement comprise entre 20 et 90°C. En particulier, le traitement enzymatique comprend successivement une étape b1) d'ajout d'une carbohydrase, avantageusement choisie parmi les pectinases, cellulases, arabanases, hémicellulases, xylanases et β-glucanases, puis une étape b2) d'ajout d'une protéase de type alcaline ou acide.

L'étape d'hydrolyse du procédé selon l'invention est très importante, puisqu'elle permet de transformer ou de « découper » les protéines natives présentes dans les graines de passiflore en peptides. Cette étape permet également avantageusement de transformer ou de « découper » les polysaccharides présents dans les graines de passiflore en oligosaccharides ou monosaccharides.

Dans une variante avantageuse du procédé, préalablement à l'étape a) les graines de passiflore sont délipidées. Avant d'être dispersées (étape a)), les graines broyées peuvent être délipidées, notamment dans de l'éthanol. L'élimination des lipides permet une meilleure efficacité des étapes ultérieures de filtration, ultrafiltration ou nanofiltration. Il est également et préférentiellement possible d'utiliser à titre de graines broyées des tourteaux de ces graines, c'est-à-dire le résidu issu de l'extraction préalable de l'huile. Cette extraction de l'huile peut être réalisée par solvant, par technique de CO₂ supercritique ou, préférentiellement, par pressage mécanique.

Lors de l'étape d), l'extrait peptidique et osidique est avantageusement récupéré par extraction de la dispersion obtenue à la fin de l'étape b, sous agitation, avantageusement à un pH compris entre 3,0 et 9,0 avantageusement à une température comprise entre 20 et 90°C.

Dans une variante avantageuse, le procédé comprend une étape supplémentaire, entre les étapes b), ou le cas échéant c), et d) de filtration ou centrifugation, éventuellement suivie d'une ultrafiltration, diafiltration, ou nanofiltration.

Les étapes de filtration ou de centrifugation, suivies notamment d'une ultrafiltration ou diafiltration sur membranes permettent d'éliminer les protéines résiduelles. Les étapes de nanofiltration permettent d'éliminer des sels minéraux ou des acides aminés libres par exemple.

Le procédé selon l'invention comprend avantageusement une étape d'ultrafiltration à 15 kDa, avantageusement entre 10 et 15 kDa, ce qui permet d'éliminer toute protéine résiduelle potentiellement allergisante.

Dans un mode de réalisation particulier selon l'invention, le procédé comprend également une étape de nanofiltration avec par exemple un seuil de coupure compris entre 100 Daltons et 300 Daltons, avantageusement entre 130 et 300 Daltons, typiquement entre 200 Daltons et 300 Daltons, pour éliminer une partie des acides aminés ou monosaccharides libres ou les sels minéraux, suite à l'étape d'ultrafiltration

Avantageusement, l'extrait peptidique et osidique peut être stabilisé par séchage, par des procédés connus de l'homme de l'art, en présence ou non d'un support de type, par exemple, maltodextrines ou fibres d'acacia (Fibregum® société CNI). La teneur en support varie typiquement selon un ratio allant de 0% à 80% en poids de support par rapport au pourcentage massique de matière sèche obtenu dans la forme liquide de l'extrait. L'extrait est préférentiellement séché par lyophilisation afin d'obtenir une poudre finale. La poudre finale comprend avantageusement 30 à 70% en poids de matière sèche de l'extrait, le complément à 100% étant le support de lyophilisation. Plus avantageusement la poudre finale comprend en poids 50% de matière sèche issue de l'extrait et 50% de support de lyophilisation.

Préférentiellement, à titre d'exemple, l'extrait peptidique et osidique peut être obtenu selon le procédé suivant :
a) mise en solution de tourteau de graines de passiflore délipidées par pressage, à 10% de matière sèche dans l'eau ;
b) hydrolyse enzymatique des carbohydrates par action d'une cellulase (Cellulyve de la société Lyven par exemple)
c) suivi d'une hydrolyse par une protéase acide (par exemple la Prolyve PAC de la société Lyven) ;
d) traitement thermique afin de dénaturer les enzymes ;
e) centrifugation, ultrafiltration et diafiltration sur membranes 15 kDa. Cette étape permet notamment d'éliminer les protéines résiduelles potentiellement allergisantes
f) nanofiltration sur membrane 200 Da. Cette étape permet notamment d'éliminer des sels minéraux ou des acides aminés libres par exemple.

La présente invention a également pour objet un extrait de graines de passiflore en particulier de graines de *Passiflore incarnata* ou de *Passiflore edulis,* avantageusement de *Passiflore edulis,* susceptible d'être obtenu par le procédé mentionné ci-dessus. Un tel extrait répond aux spécifications définies précédemment. En particulier, un tel extrait contient avantageusement 10 à 90 % en poids de peptides et 10 à 90 % en poids de sucres. Plus particulièrement, l'extrait comprend 20 à 70 %, avantageusement 30 à 65 %, en poids de peptides et 20 à 60 %, avantageusement 30 à 55 %, en poids de sucres.

L'extrait est avantageusement utilisé en tant qu'agent actif dans une composition telle qu'une composition cosmétique, dermatologique ou pharmaceutique, qui peut comprendre un ou plusieurs excipients appropriés. La composition peut en outre comprendre au moins un autre composé actif en plus de l'hydrolysat de passiflore. Cet autre composé peut être choisi parmi tous les composés et leurs équivalents fonctionnels, énoncés ci-dessous :

Cet autre composé peut être en particulier choisi parmi des actifs classiquement utilisés en dermatologie ou cosmétique tels que les émollients, les actifs hydratants, les activateurs de la synthèse de kératine, les kératorégulateurs, les kératolytiques, les agents restructurant de la barrière cutanée (activateurs de la synthèse des lipides cutanés), des agonistes PPARs (ou Peroxysome Proliferator Activated Receptor), les agonistes RXR ou LXR, les agents sébo-régulateurs, les agents anti-irritants, les agents apaisants, les agents anti-inflammatoires, les agents anti-oxydants et les agents anti-âge, les agents dépigmentants ou hypodépigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immuno-modulateurs.

Plus particulièrement, les agents cicatrisants et/ou restructurants de la barrière cutanée pouvant être utilisés en association sont avantageusement le panthénol (vitamine B5), l'arabinogalactane, l'oxyde de zinc, les céramides, le cholestérol, le squalane et les phospholipides.

Les agents sébo-régulateurs pouvant être utilisés en association sont avantageusement choisis dans le groupe constitué par les inhibiteurs de 5-alpha-réductase. Le zinc (et les dérivés de zinc tels que ses sels gluconate, salicylate et acide pyroglutamique) et la spironolactone, présentent aussi une activité sébo-suppresseur. D'autres séborégulateurs d'origine lipidique agissant sur la qualité du sébum, comme l'acide linoléique présentent également un intérêt.

L'agent anti-inflammatoire et/ou anti-irritant et/ou apaisant peut être l'arabinogalactane.

Les actifs protecteurs solaires pouvant être utilisés en association sont avantageusement des filtres et écrans solaires UVB et/ou UVA, tels les écrans ou filtres minéraux et/ou organiques connus de l'Homme du métier qui adaptera leur choix et leurs concentrations en fonction du degré de protection recherché.

Les conservateurs pouvant être utilisés en association sont par exemple ceux généralement utilisés en cosmétique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Cet autre composé peut être en particulier choisi parmi les extraits végétaux, en particulier :
- les huiles végétales telles que l'huile de soja et/ou l'huile de colza, l'huile d'avocat (WO2004/012496, WO2004/012752, WO2004/016106, WO2007/057439), l'huile de lupin et avantageusement l'huile de lupin blanc doux (WO98/47479), ou un mélange de ces huiles ;
- l'oléodistillat ou les concentrats d'huile végétale ou animale, notamment de tournesol, plus avantageusement des concentrats de tournesol linoléiques, tels que l'huile de tournesol concentrée en insaponifiables (Solinee® - WO2001/21150), commercialisée par les Laboratoires Expanscience, les huiles concentrées en insaponifiables du type huile d'avocat, de colza, de maïs utiles notamment pour leur activité hydratante et/ou émolliente, cicatrisante et/ou restructurante de la barrière cutanée, anti-inflammatoire et/ou anti-irritante et/ou apaisante ;
- Les insaponifiables de végétaux ou d'huile végétale, avantageusement des furanes d'avocat (Avocadofurane®), pouvant être obtenus par le procédé décrit dans la demande internationale WO 01/21605 , les insaponifiables d'Avocat et/ou de Soja, plus particulièrement un mélange d'insaponifiables d'Avocat furaniques et d'insaponifiables de soja, avantageusement dans un rapport respectif d'environ 1/3 - 2/3 (tel que Piasclédine®), les insaponifiables de soja (tels qu'obtenus selon le procédé décrit dans le demande internationale WO 01/51596), les insaponifiables stéroliques (typiquement des insaponifiables dont la teneur en stérols, en méthylstérols et en alcool triterpéniques est comprise entre 20 et 95% en poids, de préférence 45-65% en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;
- Les peptides ou complexes d'acides aminés végétaux, en particulier les d'avocat (tel que ceux décrits dans la demande internationale WO2005/105123), les peptides de lupin (tel que ceux décrits dans la demande internationale WO2005/102259), les peptides de quinoa (tel que ceux décrits dans la demande internationale WO2008/080974), les peptides de maca (tel que ceux décrits dans la demande internationale WO2004/112742), les peptides de soja fermenté ou non, les peptides de riz (tel que ceux décrits dans la demande internationale WO2008/009709), utiles notamment pour leur activité hydratante et/ou émolliente (avocat), kératorégulatrice (lupin, quinoa), cicatrisante et/ou restructurante de la barrière (maca, quinoa, soja), anti-inflammatoire et/ou anti-irritante et/ou apaisante (lupin, quinoa), antioxydante (avocat), anti-âge (lupin, maca), pigmentante (riz), les peptides de schizandra (tel que ceux décrits dans la demande de brevet FR 0955344), l'extrait de graines d'*Acacia macrostachya* (tels que celui décrit dans la demande de brevet FR 0958525), l'extrait de graines de *Vigna unguiculata* (tels que celui décrit dans la demande de brevet FR 0958529) ;
- Les sucres végétaux, en particulier les sucres d'avocat (tels que ceux décrits dans la demande WO2005/115421), utiles notamment pour leur propriété kératorégulatrice, cicatrisante et/ou restructurante de la barrière cutanée, anti-inflammatoire et/ou irritante et/ou apaisante ;
- Le butyle avocadate (5 alpha Avocuta®), inhibiteur de la 5-alpha réductase (WO 01/52837 et WO 02/06205) et typiquement, régulateur de la sécrétion séborrhée se trouvant augmentée dans l'acné et les pellicules ;
- Les extraits riches en polyphénols, et plus particulièrement les extraits de fruits d'avocat (tels que ceux décrits dans la demande FR 1 061 055), les extraits de feuilles de maca (tels que ceux décrits dans la demande FR 1 061 047), et les extraits de parties aériennes de *Gynandropsis gynandra* (tels que ceux décrits dans la demande FR 1 061 051),
- Le lupéol (FR 2 8 22 821, FR 2 857 596) utile notamment pour favoriser la cicatrisation ;
- Un beurre de Cupuaçu, particulièrement apprécié pour ses propriétés hydratantes.

Cet autre composé peut être en particulier choisi parmi les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline (de préférence la 2-undécyl-4,4-diméthyl-1,3-oxazoline, appelée OX-100 ou Cyclocéramide®, WO2004050052, WO2004050079, et WO2004112741). Elles sont particulièrement utiles pour leur activité anti-inflammatoire et/ou anti-irritante et/ou apaisante, antioxydante, dépigmentant, immunomodulatrice.

Toutes ces associations comprennent au moins un autre composé actif, en plus de l'extrait de graines de Passiflore, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

La composition comprenant un extrait de graines de passiflores ayant les spécifications indiquées est particulièrement destinée à une utilisation cosmétique, pharmaceutique, dermatologique, nutraceutique.

Dans le cadre d'une utilisation cosmétique, pharmaceutique, ou dermatologique, la composition sera avantageusement formulée sous la forme d'une préparation adaptée à une administration topique. La composition comprenant un extrait peptidique et osidique est particulièrement destinée à une utilisation cosmétique, pharmaceutique, ou dermatologique.

Dans le cadre d'une utilisation alimentaire, à visée nutritive ou cosmétique (« cosmet-food »), la composition sera avantageusement formulée sous la forme d'une préparation adaptée à une administration orale.

L'invention a également pour objet l'utilisation d'un extrait peptidique et osidique de graines de passiflores, ayant les spécifications décrites précédemment, pour la fabrication d'une composition cosmétique, pharmaceutique, dermatologique, d'une composition nutraceutique, ou d'un aliment fonctionnel.

Un aliment fonctionnel est un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

L'invention a ainsi pour objet un aliment fonctionnel comprenant l'extrait selon l'invention.

L'invention a aussi pour objet un extrait selon l'invention ou une composition selon l'invention pour son utilisation pour prévenir et/ou traiter :
- des troubles ou pathologies de la peau et/ou des muqueuses et/ou des phanères
- des troubles vasculaires
- des altérations du tissu adipeux,
- le stress oxydant.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

Avantageusement, la composition ou l'extrait selon l'invention peut être utilisé(e) pour la prévention et/ou le traitement des réactions, troubles ou pathologies :
- de la peau, telles que l'acné, la rosacée ou érythrocouperose, le psoriasis, les troubles vasculaires, la dermite du siège, la dermatite atopique, l'eczéma, la dermatite de contact, la dermatite irritative, la dermatite allergique, la dermite séborrhéique (croûte de lait), le psoriasis, la peau sensible, la peau réactive, la peau sèche (xérose), la peau déshydratée, la peau avec rougeur, l'érythème cutané, la peau âgée ou photoâgée, la peau photosensibilisée, la peau pigmentée (mélasma, pigmentation post-inflammatoire...), la peau dépigmentée (vitiligo), la peau avec cellulite, la peau relâchée, la peau avec vergetures, les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutisme, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

L'invention concerne également un procédé de soin cosmétique de la peau et/ou des phanères et/ou des muqueuses, en vue d'améliorer leur état et/ou leur aspect, consistant à administrer une composition ou un extrait selon la présente invention.

En particulier, le procédé de soin cosmétique permet d'affermir la peau et de diminuer l'effet « peau d'orange » avantageusement par voie topique sur la peau et/ou phanères et/ou muqueuses.

L'invention concerne un procédé de soin cosmétique de la peau, en vue d'en prévenir le vieillissement, consistant à appliquer sur la peau une composition ou un extrait selon la présente invention.

La composition ou l'extrait selon la présente invention peut également être avantageusement utilisée dans la prévention et/ou le traitement des troubles vasculaires, en particulier les rougeurs et les couperoses.

La composition ou l'extrait selon la présente invention peut également être avantageusement utilisée dans la prévention et/ou le traitement des altérations du tissu adipeux. Les altérations des tissus adipeux sont en particulier la cellulite ou l'effet « peau d'orange ». La composition selon l'invention permet d'affermir la peau.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal, comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable, ou un excipient cosmétiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

Les exemples qui suivent permettent d'illustrer l'invention.

### Exemple 1 : extrait selon l'invention

Un extrait peptidique et osidique est obtenu selon le procédé suivant :
a) mise en solution de tourteau de graines de *Passiflore edulis* délipidées par pressage, à 10% de matière sèche dans l'eau ;
b) hydrolyse enzymatique des carbohydrates par action d'une cellulase (Cellulyve de la société Lyven)
c) suivi d'une hydrolyse par une protéase acide (Prolyve PAC de la société Lyven) ;
d) traitement thermique afin de dénaturer les enzymes ;
e) centrifugation, ultrafiltration et diafiltration sur membranes 15 kDa afin d'éliminer les protéines résiduelles potentiellement allergisantes
f) nanofiltration sur membrane 200 Da afin d'éliminer des sels minéraux ou des acides aminés ou monosaccharides libres
g) récupération de l'extrait peptidique et osidique.

L'extrait peptidique et osidique liquide ainsi obtenu présente les caractéristiques suivantes :

**1 - Analyse physico-chimique (% / matière sèche totale)**

| | | |
|---|---|---|
| Extrait sec (2h, 105°C, étuve ventilée) | : | 9.7% |
| pH | : | 4.0 |
| Azote α-aminé (OPA, équivalent leucine) | : | 24% |
| Peptides (Kjeldahl, N x 6.25) | : | 56% |
| Sucres solubles (HPLC) | : | 40% dont du glucose, fructose |
| Cendres totales | : | 6% |

**2 - Profil des répartitions des masses molaires des peptides**

| | | |
|---|---|---|
| Inférieur à 130 Da | : | 26% |
| Entre 130 - 300 Da | : | 17% |
| Entre 300 - 1200 Da | : | 49% |
| Entre 1200 - 3500 Da | : | 7% |
| Supérieur à 3500 Da | : | ≤1% |

### Exemple 2 : Compositions pour application par voie topique

Plusieurs compositions pour application par voie topique sont présentées ci-dessous. L'extrait peptidique et osidique de graines de Passiflore, de l'exemple 1, peut être incorporé à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous à titre d'exemples.

**EAU NETTOYANTE HYDRATANTE**

| **Matière première / Nom commercial ou Nom INCI** | **%** |
|---|---|
| EAU PURIFIÉE | QSP 100 % |
| BIOSACCHARID GUM | De 1 à 5 % |
| BUTYLENE GLYCOL | De 1 à 5 % |
| ACIDE HYALURONIQUE | De 0 à 5 % |
| *EXTRAIT PEPTIDIQUE ET OSIDIQUE DE PASSIFLORE* | De 0,001 à 10 % |
| CONSERVATEURS | De 0 à 1 % |
| ACIDE CITRIQUE MONOHYDRATE | De 0 à 1 % |
| TROMETHAMINE | De 0 à 1 % |

**EAU NETTOYANTE PEAU SENSIBLE**

| **Matière première / Nom commercial ou Nom INCI** | **%** |
|---|---|
| CAPRYLOYL GLYCINE | De 0 à 1 % |
| LESSIVE SOUDE | De 0 à 1% |
| SEQUESTRANT | De 0 à 1 % |
| BUTYLENE GLYCOL | De 1 à 5 % |
| BETA CAROTENE | De 0 à 2 % |
| *EXTRAIT PEPTIDIQUE ET OSIDIQUE DE PASSIFLORE* | De 0,001 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

**EMULSION ANTI-AGE**

| **Matière première / Nom commercial ou Nom INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1 % |
| VITAMINE C | De 0 à 5 % |
| *ENTRAIT PEPTIDIQUE ET OSIDIQUE DE PASSIFLORE* | De 0,01 à 10 % |
| ISONONYL ISONONANOATE | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**EMULSION RESTRUCTURANTE**

| **Matière première / Nom commercial ou Nom INCI** | **%** |
|---|---|
| HYDROGENATED POLYDECENE | De 5 à 20 % |
| LAURYLGLUCOSIDE-GLYSTEARATE | De 1 à 5 % |
| DICAPRYLYL CARBONATE | De 1 à 5 % |
| GLYCEROL | De 5 à 20 % |
| CARBOPOL | De 0 à 1 % |
| GOMME XANTHANE | De 0 à 1 % |
| ACIDE ASIATIQUE | De 0 à 1 % |
| VITAMINE B5 | De 0 à 5 % |
| *EXTRAIT PEPTIDIQUE ET OSIDIQUE DE PASSIFLORE* | De 0,01 à 10 % |
| LESSIVE SOUDE | De 0 à 1 % |
| CONSERVATEURS | De 0 à 1% |
| ACIDE CITRIQUE | De 0 à 1 % |
| EAU PURIFIÉE | QSP 100 % |

**HUILE AMINCISSANTE**

| **Matière première / Nom commercial ou Nom INCI** | **%** |
|---|---|
| SOLUBILISANT | De 0 à 1 % |
| HUILE AMANDE DOUCE | De 5 à 20 % |
| CAPRYLATE / CAPRATE DE COPRAH | QSP 100 % |
| HUILE DE MACADAMIA RAFFINEE | De 5 à 20 % |
| CAPRYLO CAPRATE DE GLYCEROL | De 5 à 20 % |
| ALPHA BISABOLOL NAT | De 0 à 1 % |
| ALPHA TOCOPHEROL | De 0 à 1 % |
| EXTRAIT DE LIERRE | De 0 à 5 % |
| *EXTRAIT PEPTIDIQUE ET OSIDIQUE DE PASSIFLORE* | De 0,01 à 10 % |
| CONSERVATEUR | De 0 à 1 % |
| ESTER | De 0 à 1 % |

**LAIT pour PEAU SECHE, ATOPIQUE**

| **Matière première / Nom commercial ou Nom INCI** | % |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5 % |
| *EXTRAIT PEPTIDIQUE ET OSIDIQUE DE PASSIFLORE* | De 0,1 à 10 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**MOUSSANT**

| **Matière première / Nom commercial ou Nom INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| TENSIOACTIF 1 | De 5 à 20 % |
| TENSIOACTIF 2 | De 5 à 20 % |
| DISTEARATE DE PEG 6000 | De 1 à 5 % |
| CONSERVATEURS | De 1 à 5 % |
| *EXTRAIT PEPTIDIQUE ET OSIDIQUE DE PASSIFLORE* | De 0,001 à 10 % |
| EXTRAIT CAMOMILLE | De 1 à 5 % |
| ACIDE CITRIQUE MONOHYDRATE | De 0 à 1 % |
| SEQUESTRANT | De 0 à 1 % |
| PARFUM | De 0 à 1 % |
| LESSIVE SOUDE | De 0 à 1 % |

**SPRAY APAISANT**

| **Matière première / Nom commercial ou Nom INCI** | **%** |
|---|---|
| EAU PURIFIÉE | QSP 100 % |
| TRILAURETH-4 PHOSPHATE | De 1 à 5 % |
| DICAPRYLYL CARBONATE | De 1 à 5 % |
| BUTYLENE GLYCOL | De 1 à 5 % |
| ERYTHRITYL ESTER | De 1 à 5 % |
| HUILE VASELINE FLUIDE | De 1 à 5 % |
| BEURRE DE KARITE | De 0 à 1 % |
| HUILE VEGETALE | De 0 à 1 % |
| CONSERVATEURS | De 0 à 1% |
| LYCOPENE | De 0 à 5 % |
| *EXTRAIT PEPTIDIQUE ET OSIDIQUE DE PASSIFLORE* | De 0,01 à 10 % |
| LESSIVE SOUDE | De 0 à 1 % |
| PARFUM | De 0 à 1 % |
| GOMME XANTHANE | De 0 à 1 % |
| CARBOPOL | De 0 à 1 % |
| SEQUESTRANT | De 0 à 1 % |
| ACIDE CITRIQUE | De 0 à 1 % |

**CREME LAVANTE PURIFIANTE**

| **Matière première / Nom commercial ou Nom INCI** | **%** |
|---|---|
| EAU PURIFIEE | QSP 100 % |
| ARLATONE | De 10 à 30 % |
| COCOGLUCOSIDE | De 5 à 20 % |
| HYDROXYPROPYL GUAR | De 1 à 5 % |
| CAPRYLOYL GLYCINE | De 0 à 2 % |
| CONSERVATEURS | De 0 à 2 % |
| PARFUM | De 0 à 1 % |
| ACIDE CITRIQUE | De 0 à 1 % |
| ZINC PCA | De 0 à 1 % |
| *EXTRAIT PEPTIDIQUE ET OSIDIQUE DE PASSIFLORE* | De 0,001 à 10 % |

**EMULSION ANTI-ACNEIOUE**

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITANE | De 0 à 2 % |
| ALCOOL CETYLIQUE | De 0 à 2 % |
| ALCOOL DI-MALATE | De 5 à 20 % |
| VITAMINE E | De 0 à 1 % |
| VITAMINE B3 | De 0 à 5 % |
| ACIDE LINOLEIQUE | De 0 à 1 % |
| *EXTRAIT PEPTIDIQUE ET OSIDIQUE DE PASSIFLORE* | De 0,01 à 10 % |
| BUTYLENE GLYCOL | De 1 à 5 % |
| PIROCTOLAMINE | De 0 à 1 % |
| CONSERVATEURS | De 0 à 1% |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**EMULSION ANTI-ROUGEURS**

| **Matière première / Nom commercial ou Nom INCI** | % |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITANE | De 0 à 2 % |
| ALCOOL CETYLIQUE | De 0 à 2% |
| ALCOOL DI-MALATE | De 5 à 20 % |
| ESCULOSIDE | De 0 à 2 % |
| SOPHORA JAPONICA | De 0 à 5 % |
| VITAMINE E | De 0 à 1 % |
| *EXTRAIT PEPTIDIQUE ET OSIDIQUE DE PASSIFLORE* | De 0,01 à 10 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De0à2% |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

**SOIN REPARATEUR**

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5% |
| MONOSTEARATE DE GLYCEROL | De 1 à 5 % |
| STEARATE DE SORBITANE | De 0 à 2 % |
| ALCOOL CETYLIQUE | De 0 à 2% |
| ALCOOL DI-MALATE | De 5 à 20 % |
| VITAMINE E | De 0 à 1 % |
| COENZYME Q10 | De 0 à 2 % |
| CERAMIDE | De 0 à 5 % |
| *EXTRAIT PEPTIDIQUE ET OSIDIQUE DE PASSIFLORE* | De 0,01 à 10 % |
| BUTYLENE GLYCOL | De 1 à 5 % |
| PIROCTOLAMINE | De 0 à 1 % |
| CONSERVATEURS | De 0 à 1% |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1% |
| EAU PURIFIÉE | QSP 100 % |

**EMULSION DEPIGMENTANTE**

| **Matière première / Nom commercial ou Nom INCI** | % |
|---|---|
| ISONONYL ISONONANOATE | De 1 à 10 % |
| STEARATE D'ISOCETYLE | De 1 à 10 % |
| PEG 40 STEARATE | De 1 à 5 % |
| PEG 5 GLYCERYL STEAR | De 1 à 5 % |
| CONSERVATEURS | De 0 à 1% |
| ALCOOL CETYLIQUE C16 C18 | De 0 à 2 % |
| PPG/SMDI POLYMERE | De 0 à 1 % |
| ACIDE SALICYLIQUE | De 0 à 2 % |
| SQUALANE GEL | De 0 à 2 % |
| DIOCTYL ETHER | De 1 à 10 % |
| ALCOOL DI-MALATE | De 1 à 10 % |
| EXTRAIT DE TOURNESOL | De 1 à 10 % |
| TROMETHAMINE | De 1 à 5 % |
| BUTYLENE GLYCOL | De 1 à 10 % |
| CITRATE TRISODIQUE | De 0 à 1 % |
| SCLEROTIUM GUM | De 0 à 1 % |
| AMIDON DE RIZ | De 1 à 10 % |
| POLYACRYLAMIDE GEL | De 0 à 1 % |
| VITAMINE C | De 0 à 2 % |
| GLYCINE | De 0 à 2 % |
| PARFUM | De 0 à 1 % |
| VITAMINE E | De 0 à 2 % |
| ACIDE CITRIQUE | De 0 à 1 % |
| SEPIWHITE | De 0 à 2 % |
| *EXTRAIT PEPTIDIQUE ET OSIDIQUE DE PASSIFLORE* | De 0,01 à 10 % |
| EAU PURIFIÉE | QSP 100 % |

**STICK ROLL-ON ANTI-BACTERIEN**

| **Matière première / Nom commercial ou Nom INCI** | % |
|---|---|
| EAU PURIFIÉE | QSP 100 % |
| BUTYLENE GLYCOL | De 1 à 5 % |
| PEROXYDE DE BENZOYLE | De 0 à 2 % |
| CAPRILOYL GLYCINE | De 0 à 5 % |
| ZINC PCA | De 0 à 5 % |
| *EXTRAIT PEPTIDIQUE ET OSIDIQUE DE PASSIFLORE* | De 0,1 à 10 % |
| CARBOMERE | De 0 à 2 % |
| CONSERVATEURS | De 0 à 1% |
| ACIDE CITRIQUE | De 0 à 1 % |
| TROMETHAMINE | De 0 à 1 % |

**SOIN GOMMANT**

| **Matière première / Nom commercial ou Nom INCI** | % |
|---|---|
| ARLATONE DUO | De 5 à 20 % |
| AGENT GOMMANT | De 1 à 10 % |
| SCLEROTIUM GUM | De 1 à 10 % |
| CONSERVATEURS | De 0 à 1% |
| CAPRYLOYL GLYCINE | De 0 à 1 % |
| SOUDE | De 0 à 1% |
| *EXTRAIT PEPTIDIQUE ET OSIDIQUE DE PASSIFLORE* | De 0,01 à 10 % |
| SEQUESTRANT | De 0 à 1 % |
| ACIDE CITRIQUE | De 0 à 1 % |
| EAU PURIFIÉE | QSP 100 % |
| PARFUM | De 0 à 1 % |

**FLUIDE KERATINISANT**

| **Matière première / Nom commercial ou Nom INCI** | **%** |
|---|---|
| CETYL ALCOHOL | De 1 à 5% |
| SILICONE 345 | De 1 à 5% |
| ANTI-OXYDANT | De 0 à 1% |
| EAU PURIFIEE | QSP 100 % |
| CETRIMONIUM CHLORIDE | De 0 à 5 % |
| QUINIE | De 0 à 5 % |
| VITAMINE B5 | De 0 à 5 % |
| *EXTRAIT PEPTIDIQUE ET OSIDIQUE DE PASSIFLORE* | De 0,01 à 10 % |
| HYDROLYZED WHEAT PROTEIN | De 0 à 1 % |
| CONSERVATEUR | De 0 à 2 % |
| PARFUM | De 0 à 1 % |
| AJUSTEUR pH | De 0 à 1 % |

**SHAMPOOING ANTIPELLICULAIRE**

| **Matière première / Nom commercial ou Nom INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| *EXTRAIT PEPTIDIQUE ET OSIDIQUE DE PASSIFLORE* | De 0,01 à 10 % |
| ZINC PYRITHIONE | De 0 à 1 % |
| AJUSTEUR pH | De 0 à 1 % |
| SEQUESTRANT | De 0 à 1% |
| PARFUM | De 0 à 1 % |

**FLUIDE DEMELANT**

| **Matière première / Nom commercial ou Nom INCI** | **%** |
|---|---|
| CETEARYL ALCOHOL CETEARETH - 33 | De 1 à 5 % |
| QUATERNIUM-82 | De 0 à 2 % |
| EAU PURIFIEE | QSP 100 % |
| HYDROLYZED WHEAT PROTEIN | De 0 à 5 % |
| CONSERVATEURS | De 0 à 2 % |
| AJUSTEUR pH | De 0 à 1 % |
| PARFUM | De 0 à 1 % |
| CYSTEINE | De 0 à 5 % |
| *EXTRAIT PEPTIDIQUE ET OSIDIQUE DE PASSIFLORE* | De 0,01 à 10 % |

**LOTION CAPILLAIRE FORTIFIANTE**

| **Matière première / Nom commercial ou Nom INCI** | **%** |
|---|---|
| EAU PURIFIEE | QSP 100 % |
| METHYL PROPANEDIOL | De 5 à 20 % |
| CONSERVATEUR | De 0 à 2 % |
| AJUSTEUR pH | De 0 à 1 % |
| PARFUM | De 0 à 1 % |
| BIOTINE | De 0 à 5 % |
| VITAMINE B9 | De 0 à 5 % |
| *EXTRAIT PEPTIDIQUE ET OSIDIQUE DE PASSIFLORE* | De 0,01 à 10 % |
| BETA-SITOSTEROL | De 0 à 1 % |
| ETHYLHEXYL COCOATE | De 0 à 5% |
| PEG-40 CASTOR OIL | De 0 à 5% |

**STICK PHOTOPROTECTEUR**

| **Matière première / Nom commercial ou Nom INCI** | **%** |
|---|---|
| HUILE DE RICIN | QSP 100% |
| ALCOOL OLEIQUE | De 10 à 20% |
| HUILE PALMISTE | De 10 à 20% |
| POLYGLYCERIN-3-BEEWAX | De 10 à 20% |
| CIRE DE CANDELILLA | De 10 à 20% |
| HECTORITE | De 10 à 20% |
| TITANIUM DIOXYDE | De 0 à 5% |
| *EXTRAIT PEPTIDIQUE ET OSIDIQUE DE PASSIFLORE* | De 0,01 à 10 % |
| BEURRE DE KARITE | De 0 à 5% |
| VITAMINE E | De 0 à 1% |

**CREME SOLAIRE SPF 50+**

| **Matière première / Nom commercial ou Nom INCI** | **%** |
|---|---|
| EAU PURIFIÉE B4 | QSP 100% |
| OXYDE DE TITANE | De 10 à 20% |
| CYCLOPENTASILOXANE | De 5 à 15% |
| OCTYL PALMITATE | De 5 à 15% |
| C12-C15 ALKYL BENZOATE | De 5 à 10% |
| DECYLPENTANOATE | De 5 à 10% |
| OXYDE DE ZINC | De 5 à 10% |
| GLYCEROL | De 1 à 5% |
| PEG-45/DODECYL GLYCOL COPOLYMERE | De 1 à 5% |
| *EXTRAIT PEPTIDIQUE ET OSIDIQUE DE PASSIFLORE* | De 0,01 à 10 % |
| CHLORURE DE SODIUM | De 1 à 5% |
| DEXTRIN PALMITATE | De 1 à 2% |
| VITAMINE E | De 0 à 2% |
| CONSERVATEURS | De 0 à 2% |
| HYDROXYPROPYL GUAR | De 0 à 1% |
| ALOE VERA | De 0 à 1% |
| LESSIVE SOUDE | De 0 à 1% |
| EDTA 2 Na | De 0 à 1% |
| GLUCONATE ZINC | De 0 à 1% |

**SPRAY SOLAIRE SPF 50+**

| **Matière première / Nom commercial ou Nom INCI** | **%** |
|---|---|
| CAPRYLO CAPRATE DE GLYCEROL | De 5 à 20% |
| CYCLOPENTASILOXANE | De 10 à 20% |
| DICAPRYLYL CARBONATE | De 5 à 20% |
| TINOSORB S | De 1 à 10% |
| OXYDE DE TITANE 100 | De 10 à 20% |
| HECTORITE | De 0 à 5% |
| ALPHA TOCOPHEROL | De 0 à 2% |
| LAURYLGLUCOSIDE-GLYSTEARATE | De 0 à 10% |
| EAU PURIFIÉE B4 | QSP 100% |
| ACIDE CITRIQUE | De 0 à 2% |
| PENTYLENE GLYCOL | De 0 à 5% |
| GLYCEROL | De 0 à 5% |
| GOMME XANTHANE | De 0 à 2% |
| *EXTRAIT PEPTIDIQUE ET OSIDIQUE DE PASSIFLORE* | De 0,01 à 10 % |
| ALOE VERA | De 0 à 1% |
| GLUCONATE ZINC | De 0 à 1% |
| CONSERVATEURS | De 0 à 2% |
| TINOSORB M | De 1 à 10% |

**VERNIS POUR ONGLES FRAGILES ET CASSANTS**

| **Matière première / Nom commercial ou Nom INCI** | **%** |
|---|---|
| ACRYLATE COPOLYMER | De 15 à 30 % |
| ETHANOL | QSP 100% |
| ACETONE | De 5 à 20 % |
| *EXTRAIT PEPTIDIQUE ET OSIDIQUE DE PASSIFLORE* | De 0,01 à 5 % |

### Exemple 3 : Compositions pour administration par voie orale -

Les extraits de *Passiflore edulis* peuvent être avantageusement intégrés à des compositions orales, typiquement dans des compositions permettant l'administration de 50 mg à 200 mg d'extrait de *Passiflore edulis* par jour.

### 1/ Composition anti-vergetures sous forme de capsules molles

- EXTRAIT de *Passiflore edulis* 30 mg
- Huile d'Awara 60 mg
- Huile de colza riche en insaponifiable 300 mg
- Vitamine du groupe B (B1, B2, B3, B5, B6, B9, B12), QSP 100% des AJR
- Tocotriénols QSP 50 % AJR
- Vitamine E
- Cire d'abeille
- Lécithine de soja
- Gélatine alimentaire
- Glycérine QSP 1 capsule molle

Cette composition est administrée de 4 à 6 capsules de 500 mg par jour.

### 2/ Comprimés anti-chute cheveux

- EXTRAIT de *Passiflore edulis* 25 mg
- Extraits de céréales (blé, sarrasin, millet, épeautre)
   riche en acides aminés soufrés 200 mg
- Vitamine C QSP 50 % des AJR
- Glycosaminoglycanes issus de cartilages de poissons 200 mg
- Glucidex IT 19 (agent de compression) QSP 1 comprimé de 800 mg.

Cette composition est administrée de 5 à 8 comprimés par jour.

### 3/ Exemples en stick poudre amincissant

- EXTRAIT de *Passiflore edulis* 100 mg
- Extrait de thé riche en polyphénols 100 mg
- Extrait de raisin riche en OPC 50 mg
- Bétaglucanes d'origine végétale 100 mg
- Gomme xanthane 1 mg
- Ascorbate de sodium 0,3 mg
- Maltodextrine QSP 5 g.

Cette composition est administrée 2 fois par jour.
- EXTRAIT de *Passiflore edulis* 100 mg
- Extrait de centella asiatica 100 mg
- Magnesium, sélénium, manganèse QSP 100 % des AJR.
- Gomme xanthane 1 mg
- Ascorbate de sodium 0,3 mg
- Maltodextrine QSP 5 g.

Cette composition est administrée 2 fois par jour.

### 4/ Exemple en barre céréalière goût chocolat

- *EXTRAIT de Passiflora edulis* 200 mg
- Lycopène 6 mg,
- Astaxanthine 4 mg
- Fucoxanthine 4 mg
- Lutéine sous forme micro-encapsulée 4 mg
- Tocotriénol micro-encapsulés QSP 100 % AJR en vitamine E.
- Chocolat noir, oligofructose, sucre, sirop de fructose, cacao
   maigre en poudre, céréales croustillantes, lait écrémé en poudre,
   amandes, glycérol, sorbitol, huiles végétales, sirop de glucose,
   arôme, lait condensé sucré, lécithine de soja, mono-et
   diglycérides d'acides gras, sirop caramélisé, maltodextrine,
   sel, sorbate de potassium, alpha tocophérol. QSP une barre de 50 g.

Cette composition est administrée une fois par jour.

### 5/ Exemple en barre céréalière goût vanille

- EXTRAIT de *Passiflore edulis* 200 mg
- Extraits de céréales (blé, sarrasin, millet, épautre)
   riche en acides aminés soufrés 200 mg
- Glycosaminoglycanes issus de cartilages de poissons 200 mg
- Extrait de thé vert riche en polyphénols 200 mg
- Oligofructose, sucre, sirop de fructose, céréales
   croustillantes, lait écrémé en poudre, amandes,
   glycérol, sorbitol, huiles végétales, sirop de glucose,
   arôme, lait condensé sucré, lécithine de soja,
   mono-et diglycérides d'acides gras, sirop caramélisé,
   maltodextrine, sel, sorbate de potassium, alpha tocophérol. QSP une barre de 50 g.

Cette composition est administrée une fois par jour.

### 6/ Exemples en boisson lactée goût praliné

- EXTRAIT de *Passiflore edulis* 200 mg
- Extrait de thé vert riche en polyphénols 100 mg.
- Vitamine du groupes B (B1, B2, B3, B5, B6, B9, B12) QSP 100 % des AJR.
- Zn, Mg, Se QSP 100 % des AJR.
- Lait écrémé en poudre, arôme, fructose, blanc d'oeuf,
   noisettes, sucre, caramel, béta-carotène, gomme xanthane,
   aspartame, acésulfame de potassium, lécithine de soja,
   maltodextrine QSP un sachet de 30 g.

Cette composition est administrée une fois par jour.

### Exemple 4 : Tests d'activités biologiques de l'extrait selon l'invention

L'extrait peptidique et osidique de passiflore de l'exemple 1 est nommé ci-après dans cet exemple hydrolysat de passiflore.

### A - Screening d'activités sur Kératinocytes Humains Normaux (KHN)

Les activités biologiques de l'hydrolysat de Passiflore ont été évaluées par un test de modulation d'expression de gènes sur cultures de KHN : étude du profil d'expression sur puces Agilent Whole Genome Microarray comportant 43 376 séquences géniques.

### a - Matériel et Méthodes

Les KHN ont été mis en culture durant 24 heures, puis traités par l'hydrolysat de Passiflore à 0.05% de matière sèche. Le traitement des kératinocytes a été effectué en décalé (72h, 24h, 6h) de manière à ce que chaque point d'analyse soit cultivé durant la même durée soient 72 + 24 heures.

L'expression des gènes sélectionnés a été évaluée par RT-PCR quantitative.

Les rapports des intensités (Ri) des échantillons traités versus le contrôle ont été calculés pour chaque gène et chaque condition de traitement.

### b - Résultats

Les résultats considérés comme significatifs, sont ceux pour lesquels Ri est >2 pour chaque temps de cinétique mais aussi aux deux temps consécutifs soit 3-6h soit 6-24h. Il a pu être mis en évidence :
**Un effet anti-apoptotique,** exprimé par l'inhibition par l'hydrolysat de Passiflore du facteur de transcription ATF3, lui-même facteur transcriptionnel de gènes pro-apoptotiques de la famille des GADD, d'autres gènes pro-apoptotiques inductibles par le stress comme DDIT4, FANCE, IRS2 et sa cible FOXO3.

**Un effet activateur de la prolifération cellulaire,** via l'induction des gènes des cyclines CCNB1, CCND1 et la kinase cycline-dépendante CDK1. Cette observation est renforcée par l'induction de gènes impliqués dans divers mécanismes liés à l'activation cellulaire comme la réplication d'ADN (RFC4, TIPIN), la mitose (CDCA7, CDCA8), l'épissage (SF3A3, SRSF7), la transcription de l'ARN (POP1) ou la traduction protéique (WDR3, NOP56, MRTO4).

**Un effet activateur de la biosynthèse des stérols,** suggérant une **activité protectrice** de la couche cornée et donc de la barrière épidermique. Cela passe par l'activation des gènes : ACAT1, ACAT2, HMGCS1, HMGCR, MVD, FDPS, FDFT1, SQLE, LSS, MSMO1/SC4MOL, DHCR, EBP, DHCR7, LIPA et SOAT1.

**Un effet activateur des mécanismes anti-oxydants,** qui passe par l'induction des métallothionéines MT1A, MT1B, MT1E, MT1F, MT1G, MT1H, MT1L, MT1M, MT1X, MT2A, la stimulation de gènes de plusieurs enzymes impliquées dans la biosynthèse du glutathion: GSTA4, GSTM3, MGST2, GPX1, GPX2, GPX8, GSS, PRDX1 ou encore de la superoxyde dismutase SOD1 et de la stimulation des gènes de plusieurs facteurs de transcription codés par MAFB, ATF3 et JUN, facteurs importants pour la réponse au stress oxydatif via leur fixation ausx motifs ARE (Antioxydant Response Element) et HRE (Hypoxia Response Element).

### B - Screening d'activités sur Fibroblastes Humains Normaux (FHN)

Les activités biologiques de l'hydrolysat de Passiflore ont été évaluées par un test de modulation d'expression de gènes sur cultures de FHN. Ainsi l'expression de 46 gènes impliqués dans la biologie du derme, du remodelage des tissus conjonctifs et du vieillissement, et 46 gènes impliqués dans les fonctions clés de l'épiderme, telles que la fonction barrière en lien direct avec l'hydratation de l'épiderme, la réponse antioxydantes, ou encore la pigmentation par les mélanocytes, en présence de l'hydrolysat de passiflore dans le milieu de culture, a été étudiée par qRT-PCR.

### a - Matériel et Méthodes

L'hydrolysat de passiflore a été dilué dans le milieu de culture. Les cultures de FHN sont traitées avec 0.01% et 0.05% d'hydrolysat de passiflore pendant 24h (et par TGFβ1 molécule témoin). Au terme de l'application, les différences d'expression géniques ont été analysées par qRT-PCR. Les modifications d'expression de gènes, induites par l'hydrolysat de passiflore, sont exprimées sous forme de quantité relative (RQ) par rapport à la condition correspondant à une culture non traitée (si RQ>1 : stimulation de l'expression du gène et RQ<1 : inhibition de l'expression du gène).

### b - Résultats

### Effet activateur des mécanismes anti-oxydants

L'hydrolysat de passiflore à 0.01% induit l'expression du gène MSRA (Mitochondrial peptide méthionine sulfoxide reductase A), enzyme mitochondriale connue pour son pouvoir anti-oxydant.

De même l'hydrolysat de passiflore à 0.05% induit l'expression du gène NQO1 (NAD(H)dehydrogenase quinone 1), enzyme anti-oxydante qui possède une activité identique à la superoxyde dismutase.

Par augmentation de la MSQRA et de NQO1 ; l'hydrolysat de passiflore permettrait aux fibroblastes de derme humain d'être plus résistant vis-à-vis des dommages oxydatifs responsables du vieillissement cellulaire et donc cutané.

### Effet sur les composés de la Matrice Extra-Cellulaire

L'hydrolysat de passiflore à 0.01 et 0.05% induit l'expression du gène SDC1 codant pour le syndécan 1 qui est un protéoglycane transmembranaire et qui joue un rôle essentiel dans la cohésion des tissus.

**Tableau 1**

| | | **Hydrolysat de passiflore 0.01%** | | **Hydrolysat de passiflore 0.05%** | |
|---|---|---|---|---|---|
| ***Essais*** | ***Nom des gènes*** | ***RQ*** | ***p-value*** | ***RQ*** | ***p-value*** |
| MSRA-Hs00737165_m1 | Mitochondrial peptide méthionine sulfoxide redutase A | 1.8542 | 0.0465 | | |
| SDC₁-Hs00896424_g1 | Syndecan-1 | 1.5042 | 0.0491 | 1.5667 | 0.0388 |
| NQO₁-Hs00168547_m1 | NAD(H)dehydrogenase, quinone 1 | | | 2.0835 | 0.0414 |

### C - Screening d'activités sur Epidermes Reconstruits Mélanisés

Les activités biologiques de l'hydrolysat de Passiflore ont été évaluées par un test de modulation d'expression de gènes sur épidermes reconstruits mélanisés. Ainsi l'expression de 46 gènes impliqués dans la biologie du derme, du remodelage des tissus conjonctifs et du vieillissement, et 46 gènes impliqués dans les fonctions clés de l'épiderme, telles que la fonction barrière en lien direct avec l'hydratation de l'épiderme, la réponse anti-oxydantes, ou encore la pigmentation par les mélanocytes, en présence de l'hydrolysat de passiflore dans le milieu de culture, a été étudiée par qRT-PCR.

### a - Matériel et Méthodes

L'hydrolysat de passiflore a été dilué dans le milieu de culture. Les épidermes sont traitées avec 0.01% et 0.05% d'hydrolysat de passiflore pendant 24h (et par TGFβ1 molécule témoin). Au terme de l'application, les différences d'expression géniques ont été analysées par qRT-PCR. Les modifications d'expression de gènes, induites par l'hydrolysat de passiflore, sont exprimées sous forme de quantité relative (RQ) par rapport à la condition correspondant à une culture non traitée (si RQ>1 : stimulation de l'expression du gène et RQ<1 : inhibition de l'expression du gène).

### b - Résultats

### Effet sur les gènes impliqués dans la cohésion derme / épiderme

L'hydrolysat de Passiflore à 0.05% induit l'expression de LAMC2, gène codant pour la laminine C2 ; l'hydrolysat de passiflore pourrait ainsi contribuer au renfort de la jonction dermo-épidermique et donc favoriser la fermeté de la peau.

### Effet sur les gènes impliqués dans la fonction barrière de l'épiderme

L'hydrolysat de passiflore à 0.05% induit l'expression du gène codant pour l'involucrine (IVL), protéine structurelle de l'enveloppe cornée, impliquée dans les étapes d'initiation et de maturation des cornéocytes et joue donc un rôle essentiel dans la fonction barrière de l'épiderme. Il stimule également l'expression du gène de la filaggrine (FLG), impliquée également dans l'hydratation et la fonction barrière.

### Effet sur d'autres gènes

L'hydrolysat de passiflore diminue l'expression de MC1R, impliqué dans le processus de mélanogénèse, de PTGS2, impliqué dans la réponse inflammatoire et BIRC5, facteur anti-apoptotique marqueur des cellules souches.

**Tableau 2**

| | | **Hydrolysat de passiflore 0.01%** | | **Hydrolysat de passiflore 0.05%** | |
|---|---|---|---|---|---|
| ***Essais*** | ***Nom des gènes*** | ***RQ*** | ***p-value*** | ***RQ*** | ***p-value*** |
| LACMC2 - Hs01043711_m1 | Laminin subunit gamma-2 | | | 2.7769 | 0.026 |
| IVL-Hs00846307_s1 | Involucrin | | | 1.4151 | 0.0048 |
| MC1R-Hs00267167_s1 | Melanocyte-stimuling hormone receptor | | | 0.7131 | 0.0194 |
| PTGS2 - Hs01573476_g1 | Prostaglandin G/H synthase 2 | | | 0.5185 | 0.0438 |
| BIC5 - Hs00977611_g1 | Baculoviral IAP repeat-containing protein 5 (survivin) | | | 0.5122 | 0.0007 |
| NTRK2 - Hs01093103_m1 | BDNF/NT-3 growth factors receptor | | | 0.1707 | 0.0359 |
| FLG - Hs00863478_g1 | Filaggrin | 0.7257 | 0.01555 | | |

### D. Effet sur la lipolyse du tissu adipeux humain

Le tissu adipeux blanc humain exerce une fonction métabolique fondamentale en fournissant aux autres tissus de l'organisme des molécules énergétiques sous forme d'acides gras libérés par le processus de lipolyse adipocytaire. L'adipocyte mobilise ses réserves énergétiques par hydrolyse des triglycérides stockés en acides gras et glycérol. Les acides gras ainsi libérés dans le sang sont utilisables par les autres tissus à des fins énergétiques.

L'effet de l'hydrolysat de passiflore sur la lipolyse adipocytaire a été évalué par le dosage du glycérol libéré lors de l'hydrolyse des triglycérides stockés dans les adipocytes matures humains cultivés en 3 dimensions.

### a) Matériel et méthodes

Des adipocytes matures, isolés à partir de biopsies de tissu adipeux sous-cutané de quatre donneurs femmes normopondérées ou en surpoids, ont été incubés en présence de l'hydrolysat de passiflore pendant 4 heures à 37°C. Le glycérol libéré a été dosé par une méthode colorimétrique, les valeurs obtenues ont été normalisées à la quantité d'ADN. Les résultats ont été comparés statistiquement au moyen d'une analyse de variance à un facteur suivie d'un test de Dunnett.

### b) Résultats

Les résultats sont reportés dans le tableau suivant :

**Tableau 3**

| | Glycérol (données normalisées ; moyenne±SD) | |
|---|---|---|
| Contrôle | 1,00 | |
| Hydrolysat de passiflore 0,005% | 1,69 ± 0,23 | p<0,05 |

L'hydrolysat de passiflore à 0,005% a significativement stimulé la libération de glycérol montrant un effet activateur de la lipolyse adipocytaire.

### E. Activité anti-tyrosinase

La tyrosinase est une enzyme clé dans la synthèse de mélanine. Des inhibiteurs spécifiques de cette enzyme peuvent conduire à une inhibition de la synthèse de mélanine et ainsi induire des effets dépigmentants.

L'effet de l'hydrolysat de passiflore a été évalué sur l'activité de la tyrosinase extraite de mélanocytes humains (test acellulaire).

### a) Matériel et méthodes

L'hydrolysat de passiflore et la référence de l'essai (acide kojique) ont été mis en présence de l'extrait enzymatique (tyrosinase) et incubés pendant 10 minutes sur glace. A la fin de l'incubation, le substrat L-DOPA à 2 mM a été ajouté et les échantillons ont été incubés 1 heure à 37°C. L'activité enzymatique a été évaluée par colorimétrie (lecture de la densité optique à 540 nm). Les résultats ont été exprimés en pourcentage d'activité tyrosinase et comparés statistiquement à l'aide d'un test t de Student.

### b) Résultats

Les résultats sont reportés dans le tableau suivant :

**Tableau 4**

| | **Activité tyrosinase** (% Témoin) | ***P*** | CI50 |
|---|---|---|---|
| Contrôle | **100** | - | - |
| Acide kojique 0,0625 mM | **95** | *Ns* | 0,76 mM |
| Acide kojique 0,125 mM | **93** | *Ns* | |
| Acide kojique 0,25 mM | **88** | *p<0,05* | |
| Acide kojique 0,5 mM | **66** | *p<0,001* | |
| Acide kojique 1 mM | **36** | *p<0,001* | |
| Hydrolysat de passiflore 0,005% | **101** | *Ns* | 0,16% |
| Hydrolysat de passiflore 0,01% | **97** | *Ns* | |
| Hydrolysat de passiflore 0,05% | **83** | *p<0,05* | |
| Hydrolysat de passiflore 0,1% | **70** | *p<0,01* | |
| Hydrolysat de passiflore 0,5% | **0** | *p<0,001* | |
| Hydrolysat de passiflore 1% | **-3** | *p<0,001* | |

L'hydrolysat de passiflore a inhibé de façon nette et concentration-dépendante l'activité enzymatique de la tyrosinase (CI50 de 0,16%).

### F. Protection contre le stress oxydant

Le stress oxydant, qu'il soit induit de façon endogène ou exogène (tabagisme, pollution atmosphérique, rayonnements UV) est responsable de nombreux désordres cutanés. En effet, via la production de radicaux libres, le stress oxydant peut altérer les différents constituants des cellules de la peau : protéines, lipides, ADN des noyaux et des mitochondries. Un effet anti-oxydant de l'hydrolysat de passiflore a été confirmé en évaluant, d'une part, sa capacité à moduler la production de radicaux libres et d'autre part, son effet protecteur contre la peroxydation des lipides dans des cellules soumises à un stress oxydant.

### 1. Effet sur la production d'espèces réactives de l'oxygène dans des kératinocytes

L'action anti-oxydante de l'hydrolysat de passiflore vis-à-vis d'un stress oxydatif généré par de l'eau oxygénée (H₂O₂) dans des kératinocytes a été évaluée par mesure de la quantité d'espèces réactives de l'oxygène produites. Ce test est basé sur l'utilisation d'une sonde (DCFH-DA) qui est dégradée et fluoresce au contact des ROS (Reactive Oxygen Species ou Espèces Réactives de l'Oxygène). La fluorescence émise sera donc proportionnelle à la quantité de ROS produits par la cellule en réponse au stress oxydatif.

### a) Matériel et méthodes

Des kératinocytes humains normaux ont été pré-incubés pendant 24 heures en présence d'hydrolysat de passiflore, ou des molécules de référence : Quercétine à 10µM ou Vitamine C à 500µM. Les cellules ont ensuite été incubées pendant 1 heure en présence de la sonde DCFH-DA puis traitées par l'H₂O₂ à 100µM pendant 20 minutes, toujours en présence de l'hydrolysat de passiflore ou des molécules de référence. Le dosage des ROS a été réalisé par mesure de la fluorescence émise. Les résultats ont été normalisés par le nombre de cellules vivantes déterminé par un test d'incorporation du rouge neutre réalisé en parallèle. La quantité de ROS émis est donc exprimée en densité de fluorescence (DFU) / densité optique issue du test au rouge neutre (DO₅₄₀). Les résultats ont été analysés statistiquement par une analyse de variance à un facteur suivie d'un test de Tukey.

### b) Résultats

Les résultats sont reportés dans le tableau suivant :

**Tableau 5**

| | **Quantité de ROS** DFU/ DO₅₄₀ (moyenne ± ecart-type) | Augmentation par rapport au contrôle | Inhibition par rapport au témoin H₂O₂ |
|---|---|---|---|
| Contrôle | 62132.821 ± 10278.568 | | |
| Témoin H202 (stress) | 287593.309 ± 45380.022 | +363%p<0,001 | |
| Quercétine (référence) | 84549.570 ± 9476.724 | | -71% p<0,001 |
| Vitamine C (référence) | 35997.820 ± 10389.067 | | -87% p<0,001 |
| Hydrolysat de passiflore 0,01% | 98585.727 ± 7972.591 | | -66% p<0,001 |
| Hydrolysat de passiflore 0,05% | 56063.620 ± 3540.141 | | -81% p<0,001 |
| Hydrolysat de passiflore 0,1% | 45893.532 ± 3120.083 | | -84% p<0,001 |

L'hydrolysat de passiflore a fortement et de façon hautement significative inhibé la production de ROS induits par le stress H₂O₂.

### 2. Effet protecteur vis-à-vis de la peroxydation lipidique

L'effet protecteur de l'hydrolysat de passiflore vis-à-vis de la peroxydation lipidique a été évalué dans des cellules Jurkat irradiées aux UV.

### a) Matériel et méthodes

Les cellules Jurkat ont été incubées pendant 45 minutes en présence ou non de l'hydrolysat de passiflore ou de la référence (BHT à 50µM) et en présence de la sonde fluorescente spécifique pour la mesure des peroxydes lipidiques (C11-fluor). Cette sonde fluorescente est un analogue de lipide qui s'intègre aux membranes et dont l'intensité de fluorescence diminue lorsqu'elle est peroxydée. Les cellules ont ensuite été irradiées par les UV (UVB 240mJ/cm² + UVA 3,5J/cm²) en présence de l'hydrolysat de passiflore ou de la référence. A la fin de l'irradiation, les cellules ont été incubées pendant 30 minutes à 37°C avant d'être analysée par cytométrie en flux. Les résultats obtenus en intensité de fluorescence ont été exprimés en % du témoin irradié.

### b) Résultats

Les résultats sont reportés dans le tableau suivant :

**Tableau 6**

| | **Quantité de peroxydes lipidiques** (% du témoin irradié) | Protection (%) |
|---|---|---|
| Contrôle (non irradié) | **46** | 100 |
| Témoin irradié (UV) | **100** | 0 |
| UV + BHT | **83** | 31 |
| UV + Hydrolysat de passiflore 0,05% | **87** | 24 |

L'hydrolysat de passiflore à 0,05% a protégé les cellules de la peroxydation lipidique induite par l'irradiation UV (24% de protection).

## Revendications

1. Composition comprenant un extrait peptidique et osidique de graines de Passiflore, en particulier de graines de *Passiflore incarnata* ou de *Passiflore edulis,* avantageusement de *Passiflore edulis,* en tant que principe actif, et le cas échéant un excipient approprié, **caractérisée en ce que** l'extrait peptidique et osidique est obtenu par hydrolyse enzymatique en présence d'au moins une protéase et au moins une carbohydrase.

2. Composition selon la revendication 1, **caractérisée en ce que** dans l'extrait peptidique et osidique, les peptides ont une taille inférieure à 3500 Da.

3. Composition selon l'une quelconque des revendications précédentes, **caractérisé en ce que** dans l'extrait peptidique et osidique, le ratio massique peptides/sucres est supérieur à 0,75 et de préférence compris entre 1 et 2.

4. Composition selon la revendication 3, **caractérisé en ce que** l'extrait peptidique et osidique comprend 20 à 70 %, avantageusement 30 à 65 %, typiquement 55%, en poids de peptides.

5. Composition selon la revendication 3, **caractérisé en ce que** l'extrait peptidique et osidique comprend 20 à 60 %, avantageusement 30 à 55 %, typiquement 40%, en poids de sucres.

6. Composition selon l'une quelconque des revendications précédentes, comprenant 0,001 à 10% en poids, avantageusement 0,01 à 5%, dudit extrait peptidique et osidique de graines de Passiflore, le poids de l'extrait étant exprimé en extrait sec, par rapport au poids total de la composition.

7. Composition selon l'une quelconque des revendications précédentes, comprenant en outre un autre principe actif, en plus de l'extrait peptidique et osidique de graines de passiflore, ledit autre principe actif étant avantageusement choisi parmi
- les émollients, les actifs hydratants, les activateurs de la synthèse de kératine, les kératorégulateurs, les kératolytiques, les agents restructurant de la barrière cutanée (activateurs de la synthèse des lipides cutanés), des agonistes PPARs (ou Peroxysome Proliferator Activated Receptor), les agonistes RXR ou LXR, les agents sébo-régulateurs, les agents anti-irritants, les agents apaisants, les agents anti-inflammatoires, les agents anti-oxydants et les agents anti-âge, les agents dépigmentants ou hypodépigmentants, les agents pigmentants, les agents lipolytiques ou inhibiteurs de la lipogénèse ou encore les agents anti-cellulite ou amincissants, les filtres et écrans solaires minéraux ou organiques, les composés antifongiques, les conservateurs, les agents anti-bactériens, les pré et probiotiques, les antibiotiques, les immuno-modulateurs ;
- les extraits végétaux ; et
- les oxazolines.

8. Procédé de préparation d'un extrait peptidique et osidique des graines de passiflore, en particulier de graines de *Passiflore incarnata* ou de *Passiflore edulis,* avantageusement de *Passiflore edulis,* comprenant au moins une étape d'hydrolyse enzymatique en présence d'au moins une protéase et au moins une carbohydrase.

9. Procédé selon la revendication 8, **caractérisé en ce qu'**il comprend les étapes successives suivantes :
a) dispersion en phase aqueuse des graines broyées ; puis
b) traitement enzymatique, par ajout d'au moins une protéase et d'au moins une carbohydrase, de la dispersion aqueuse obtenue à l'étape a), puis
c) le cas échéant, traitement thermique pour dénaturer les enzymes, et
d) récupération de l'extrait peptidique et osidique à la fin de l'étape b) ou c).

10. Procédé selon la revendication 9, **caractérisé en ce que** le traitement enzymatique comprend successivement une étape b1) d'ajout d'une carbohydrase, avantageusement choisie parmi les pectinases, cellulases, arabanases, hémicellulases, xylanases et β-glucanases, puis une étape b2) d'ajout d'une protéase.

11. Procédé selon l'une quelconque des revendications 9 à 10, **caractérisé en ce que** préalablement à l'étape a) les graines sont délipidées.

12. Procédé selon l'une quelconque des revendications 9 à 11, **caractérisé en ce qu'**il comprend une étape supplémentaire, entre les étapes b), ou le cas échéant c), et d) de filtration ou centrifugation, éventuellement suivie d'une ultrafiltration, diafiltration, ou nanofiltration.

13. Procédé selon la revendication 12, **caractérisé en ce qu'**il comprend une étape d'ultrafiltration à 15 kDa.

14. Extrait peptidique et osidique de graines de passiflore, en particulier de graines de *Passiflore incarnata* ou de *Passiflore edulis,* avantageusement de *Passiflore edulis,* susceptible d'être obtenu par le procédé selon l'une quelconque des revendications 8 à 13, contenant 10 à 90 % en poids de peptides et 10 à 90 % en poids de sucres.

15. Extrait selon la revendication 14, contenant 20 à 70 %, avantageusement 30 à 65 %, typiquement 55%, en poids de peptides et 20 à 60 %, avantageusement 30 à 55 %, typiquement 40%, en poids de sucres.

16. Extrait selon la revendication 14 ou 15 ou composition selon l'une quelconque des revendications 1 à 7 pour son utilisation pour prévenir et/ou traiter :
- des troubles ou pathologies de la peau et/ou des muqueuses et/ou des phanères
- des troubles vasculaires
- la cellulite
- les désordres cutanés dus au stress oxydant.

17. Procédé de soin cosmétique de la peau et/ou des phanères et/ou des muqueuses, en vue d'améliorer leur aspect, consistant à administrer une composition cosmétique telle que définie selon l'une quelconque des revendications 1 à 7 ou un extrait tel que défini selon l'une quelconque des revendications 14 ou 15.

## Patentansprüche

1. Zusammensetzung, umfassend einen Peptid- und Zuckerextrakt aus Passionsblumensamen, insbesondere aus Samen von *Passiflora incarnata* oder von *Passiflora edulis,* in vorteilhafter Weise von *Passiflora edulis,* als Wirkstoff, und gegebenenfalls einen geeigneten Hilfsstoff, **dadurch gekennzeichnet, dass** der Peptid- und Zuckerextrakt durch enzymatische Hydrolyse bei Anwesenheit von mindestens einer Protease und mindestens einer Carbohydrase gewonnen wird.

2. Zusammensetzung nach Anspruch 1, **dadurch gekennzeichnet, dass** die Peptide in dem Peptid- und Zuckerextrakt eine Größe von unter 3500 Da haben.

3. Zusammensetzung nach einem der vorangehenden Ansprüche, **dadurch gekennzeichnet, dass** das Massenverhältnis Peptide/Zucker in dem Peptid- und Zuckerextrakt größer als 0,75 und vorzugsweise zwischen 1 und 2 inklusive ist.

4. Zusammensetzung nach Anspruch 3, **dadurch gekennzeichnet, dass** der Peptid- und Zuckerextrakt 20 bis 70 Gew.-%, in vorteilhafter Weise 30 bis 65 Gew.-%, typischerweise 55 Gew.-% Peptide umfasst.

5. Zusammensetzung nach Anspruch 3, **dadurch gekennzeichnet, dass** der Peptid- und Zuckerextrakt 20 bis 60 Gew.-%, in vorteilhafter Weise 30 bis 55 Gew.-%, typischerweise 40 Gew.-% Zucker umfasst.

6. Zusammensetzung nach einem der vorangehenden Ansprüche, umfassend 0,001 bis 10 Gew.-%, in vorteilhafter Weise 0,01 bis 5 %, des Peptid- und Zuckerextrakts aus Passionsblumensamen, wobei das Gewicht des Extrakts in Trockenextrakt in Bezug zum Gesamtgewicht der Zusammensetzung ausgedrückt ist.

7. Zusammensetzung nach einem der vorangehenden Ansprüche, umfassend ferner einen anderen Wirkstoff zusätzlich zum Peptid- und Zuckerextrakt aus Passionsblumensamen, wobei der andere Wirkstoff in vorteilhafter Weise ausgewählt ist aus
- den Weichmachern, den feuchtigkeitsspendenden Wirkstoffen, den Aktivatoren der Keratinsynthese, den Keratinregulatoren, den Keratolytika, den restrukturierenden Wirkstoffen der Hautbarriere (Aktivatoren der Synthese der Hautlipide), PPARs-Agonisten (oder Peroxysome Proliferator Activated Receptor), den RXR- oder LXR-Agonisten, den talgregulierenden Wirkstoffen, den Wirkstoffen gegen Reizungen, den besänftigenden Wirkstoffen, den entzündungshemmenden Wirkstoffen, den antioxidierenden Wirkstoffen und den Anti-Age-Wirkstoffen, den depigmentierenden oder hypodepigmentierenden Wirkstoffen, den pigmentierenden Wirkstoffen, den lipolytischen Wirkstoffen oder Hemmern der Lipogenese oder auch den Anti-Cellulite- oder figurverbessernden Wirkstoffen, den mineralischen oder organischen Sonnenfiltern und - schilden, den fungiziden Verbindungen, den Konservierungsstoffen, den antibakteriellen Wirkstoffen, den Prä- und Probiotika, den Antibiotika, den Immunmodulatoren,
- den Pflanzenextrakten und
- den Oxazolinen.

8. Herstellungsverfahren eines Peptid- und Zuckerextrakts aus Passionsblumensamen, insbesondere aus Samen von *Passiflora incarnata* oder von *Passiflora edulis,* in vorteilhafter Weise von *Passiflora edulis,* umfassend mindestens einen enzymatischen Hydrolyseschritt bei Anwesenheit von mindestens einer Protease und mindestens einer Carbohydrase.

9. Verfahren nach Anspruch 8, **dadurch gekennzeichnet, dass** es die folgenden aufeinanderfolgenden Schritte umfasst:
a) Dispersion in wässriger Phase der zerkleinerten Samen, dann
b) enzymatische Behandlung, durch Hinzufügen von mindestens einer Protease und mindestens einer Carbohydrase, der wässrigen Dispersion aus Schritt a), dann
c) gegebenenfalls thermische Behandlung zwecks Denaturieren der Enzyme, und
d) Gewinnen des Peptid- und Zuckerextrakts am Ende von Schritt b) oder c).

10. Verfahren nach Anspruch 9, **dadurch gekennzeichnet, dass** die enzymatische Behandlung aufeinanderfolgend einen Schritt b1) des Hinzufügens einer Carbohydrase, in vorteilhafter Weise ausgewählt aus den Pektinasen, Cellulasen, Arabanasen, Hemicellulasen, Xylanasen und β-Glucanasen, dann einen Schritt b2) des Hinzufügens einer Protease umfasst.

11. Verfahren nach einem der Ansprüche 9 bis 10, **dadurch gekennzeichnet, dass** die Samen vor dem Schritt a) delipidiert werden.

12. Verfahren nach einem der Ansprüche 9 bis 11, **dadurch gekennzeichnet, dass** es einen zusätzlichen Filtrations- oder Zentrifugierungsschritt zwischen den Schritten b) oder gegebenenfalls c) und d), eventuell gefolgt von einer Ultrafiltration, Diafiltration oder Nanofiltration, umfasst.

13. Verfahren nach Anspruch 12, **dadurch gekennzeichnet, dass** es einen Ultrafiltrationsschritt auf 15 kDa umfasst.

14. Peptid- und Zuckerextrakt aus Passionsblumensamen, insbesondere aus Samen von *Passiflora incarnata* oder von *Passiflora edulis,* in vorteilhafter Weise von *Passiflora edulis,* gewinnbar nach dem Verfahren nach einem der Ansprüche 8 bis 13, enthaltend 10 bis 90 Gew.-% Peptide und 10 bis 90 Gew.-% Zucker.

15. Extrakt nach Anspruch 14, enthaltend 20 bis 70 Gew.-%, in vorteilhafter Weise 30 bis 65 Gew.-%, typischerweise 55 Gew.-% Peptide und 20 bis 60 Gew.-%, in vorteilhafter Weise 30 bis 55 Gew.-%, typischerweise 40 Gew.-% Zucker.

16. Extrakt nach Anspruch 14 oder 15 oder Zusammensetzung nach einem der Ansprüche 1 bis 7 für seine Verwendung zur Vorbeugung und/oder Behandlung:
- von Störungen oder Pathologien der Haut und/oder der Schleimhäute und/der der Hautanhangsgebilde,
- von Gefäßstörungen,
- von Cellulite,
- von Hautunregelmäßigkeiten aufgrund von oxidativem Stress.

17. Verfahren für die kosmetische Pflege der Haut und/oder der Hautanhangsgebilde und/oder der Schleimhäute zwecks Verbesserung ihres Aussehens, das darin besteht, eine kosmetische Zusammensetzung nach einem der Ansprüche 1 bis 7 oder einen Extrakt nach einem der Ansprüche 14 oder 15 zu verabreichen.

## Claims

1. A composition comprising a peptide and sugar extract of passionflower seeds, particularly *Passiflora incarnata* seeds or *Passiflora edulis* seeds, advantageously *Passiflora edulis* seeds, as active agent, and if need be a suitable excipient, **characterized in that** the peptide and sugar extract is obtained by enzymatic hydrolysis, in the presence of at least one protease and at least one carbohydrase.

2. The composition according to claim 1, **characterized in that** in the peptide and sugar extract, the peptides have a size less than 3500 Da.

3. The composition according to any one of the preceding claims, **characterized in that** in the peptide and sugar extract, the weight ratio of peptides/sugars is greater than 0.75 and preferably comprised between 1 and 2.

4. The composition according to claim 3, **characterized in that** the peptide and sugar extract comprises 20% to 70%, advantageously 30% to 65%, typically 55%, by weight of peptides.

5. The composition according to claim 3, **characterized in that** the peptide and sugar extract comprises 20% to 60%, advantageously 30% to 55%, typically 40%, by weight of sugars.

6. The composition according to any one of the preceding claims, comprising 0.001% to 10% by weight, advantageously 0.01% to 5%, of said peptide and sugar extract of passionflower seeds, the weight of the extract being expressed in dry extract, in relation to the total weight of the composition.

7. The composition according to any one of the preceding claims, further comprising one other active agent, in addition to the peptide and sugar extract of passionflower seeds, said one other active agent being advantageously selected from:
- emollients, moisturizing active agents, keratin-synthesis activators, keratoregulators, keratolytics, agents that restructure the cutaneous barrier (activators of synthesis of cutaneous lipids), peroxisome proliferator-activated receptor (PPAR) agonists, RXR or LXR agonists, sebum-regulating agents, anti-irritant agents, soothing agents, anti-inflammatory agents, antioxidants and anti-aging agents, depigmenting or hypodepigmenting agents, pigmenting agents, lipolytic agents or lipogenesis inhibitors or anti-cellulite or slimming agents, inorganic or organic sun filters and screens, antifungal compounds, preservatives, antibacterial agents, prebiotics and probiotics, antibiotics, immunomodulators;
- plant extracts; and
- oxazolines.

8. A process for preparing a peptide and sugar extract of passionflower seeds, particularly *Passiflora incarnata* seeds or *Passiflora edulis* seeds, advantageously *Passiflora edulis* seeds, comprising at least one enzymatic hydrolysis step, in the presence of at least one protease and at least one carbohydrase.

9. The process according to claim 8, **characterized in that** it comprises the following successive steps:
a) dispersion in an aqueous phase of grinded seeds, then
b) enzymatic treatment, by adding at least one protease and at least one carbohydrase, of the aqueous dispersion obtained in step a), then
c) if need be, heat treatment in order to denature the enzymes, and
d) collection of the peptide and sugar extract at the conclusion of step b) or c).

10. The process according to claim 9, **characterized in that** the enzymatic treatment successively comprises a step b1) of adding a carbohydrase, advantageously selected from pectinases, cellulases, arabanases, hemicellulases, xylanases and β-glucanases, then a step b2) of adding a protease.

11. The process according to any one of claims 9 to 10, **characterized in that** prior to step a) the seeds are defatted.

12. The process according to any one of claims 9 to 11, **characterized in that** it comprises an additional step, between steps b), or if need be c), and d) of filtration or centrifugation, optionally followed by ultrafiltration, diafiltration or nanofiltration.

13. The process according to claim 12, **characterized in that** it comprises a step of 15 kDa ultrafiltration.

14. A peptide and sugar extract of passionflower seeds, particularly *Passiflora incarnata* seeds or *Passiflora edulis* seeds, advantageously *Passiflora edulis* seeds, able to be obtained by the process according to any one of claims 8 to 13, containing 10% to 90% by weight of peptides and 10% to 90% by weight of sugars.

15. The extract according to claim 14, containing 20% to 70%, advantageously 30% to 65%, typically 55%, by weight of peptides and 20% to 60%, advantageously 30% to 55%, typically 40%, by weight of sugars.

16. The extract according to claim 14 or 15 or composition according to any one of claims 1 to 7 for use to prevent and/or treat:
- disorders or pathological conditions of the skin and/or the mucous membranes and/or the skin appendages,
- vascular disorders,
- cellulite,
- skin disorders due to oxidative stress.

17. A cosmetic care process for the skin and/or the skin appendages and/or the mucous membranes, with a view to improving their appearance, consisting in administering a cosmetic composition as defined according to any one of claims 1 to 7 or an extract as defined according to any one of claims 14 or 15.
